# EUROPEAN PATENT SPECIFICATION

(11) **EP 0 768 839 B1**
(45) Date of publication and mention of the grant of the patent: **23.12.1998**
(21) Application number: 93919495.7
(22) Date of filing: 03.09.1993
(51) Int. Cl.: A61B 17/12, A61B 17/04, A61B 1/00

(54) **ENDOSCOPE HAVING A DEVICE FOR USE IN TYING KNOTS**
ENDOSKOP MIT EINER VORRICHTUNG ZUR ANWENDUNG BEIM BINDEN VON KNOTEN
ENDOSCOPE MUNI D'UN DISPOSITIF UTILISE POUR FAIRE DES NOEUDS

(30) Priority: 04.09.1992 GB 9218752
(43) Date of publication of application: 23.04.1997
(73) Proprietor: UNIVERSITY COLLEGE LONDON, London WC1E 6BT (GB)
(72) Inventor: SWAIN, Paul, London NW3 1TN (GB); GONG, Feng Shropshire House, London WC1E 6JA (GB); BROWN, Geoffrey, John, London E10 7AN (GB); MILLS, Timothy, Noel Flat 4, London WC1N 3PA (GB)
(74) Representative: Boon, Graham Anthony
(86) International application number: GB9301859
(87) International publication number: WO9405220

(56) References cited:
- DE-A- 2 900 265
- GB-A- 2 247 841

## Description

This invention relates to a device for use in tying knots during surgery carried out using an endoscope, notably a flexible endoscope.

It has hitherto proved difficult to tie knots during flexible endoscopy. In effect, the surgeon is operating down a single longitudinally extending channel, and in endeavouring to tie a knot the surgeon encounters the problem that an effective knot generally requires the application of a force along a direction transverse to the channel of the endoscope. The present invention aims to provide a solution to that problem.

According to the present invention there is provided an endoscope having a distal end and a proximal end, and a thread guide device in the form of an annular member which surrounds the said distal end without impairing the field of view of the endoscope, the thread guide device having a pair of thread guide means which are laterally spaced from one another, the device permitting a knot formed by threads which have passed through the thread guide means to be viewed by the endoscope.

In the accompanying diagrammatic drawings:
Figure 1 is a perspective view of a thread guide device according to the invention mounted on the distal end of an endoscope;
Figure 2 shows the device and endoscope of Figure 1, being used to tie a half hitch knot; and
Figure 3 shows the device and endoscope of Figure 1 being used to tie a self-lock slipknot.

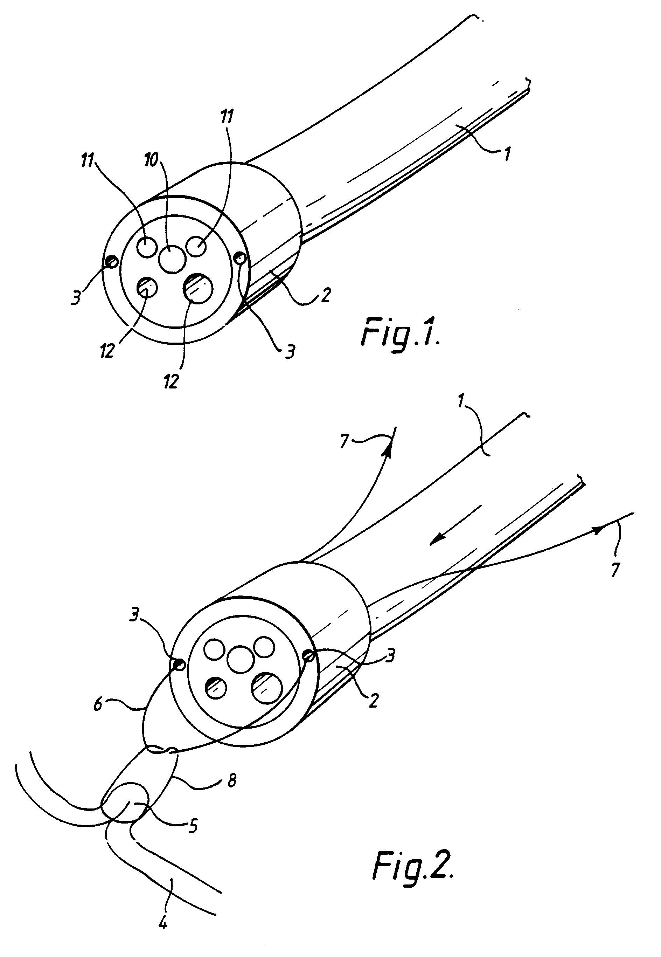

Figure 1 shows an endoscope 1 having a thread guide device 2 mounted on the distal end thereof. The endoscope itself can be conventional, and the type illustrated has an image bundle 10, two illumination bundles 11, and two operating channels 12. It must be emphasised, however, that other forms of endoscope could be used instead.

The device 2 takes the form of a short tube of circular cross-section, which surrounds the distal end portion of the endoscope 1. The device 2 may be mounted on the endoscope 1 either as a push fit, by providing the device 2 with an internal thread to engage the external thread provided on some conventional endoscopes, or in any other appropriate fashion.

The device 2 has a pair of thread guide passages 3 which extend parallel to the longitudinal axis of the endoscope 1 and device 2 from one end of the device to the other. The passages are sufficiently large in cross-section to permit a thread of the type normally used in surgery to pass therethrough. As illustrated, the two passages 3 are preferably arranged substantially at 180° with respect to one another about the axis of the device 2, but though this is desirable from the point of view of enabling the maximum force to be exerted on the knot which is being tied, it is not essential.

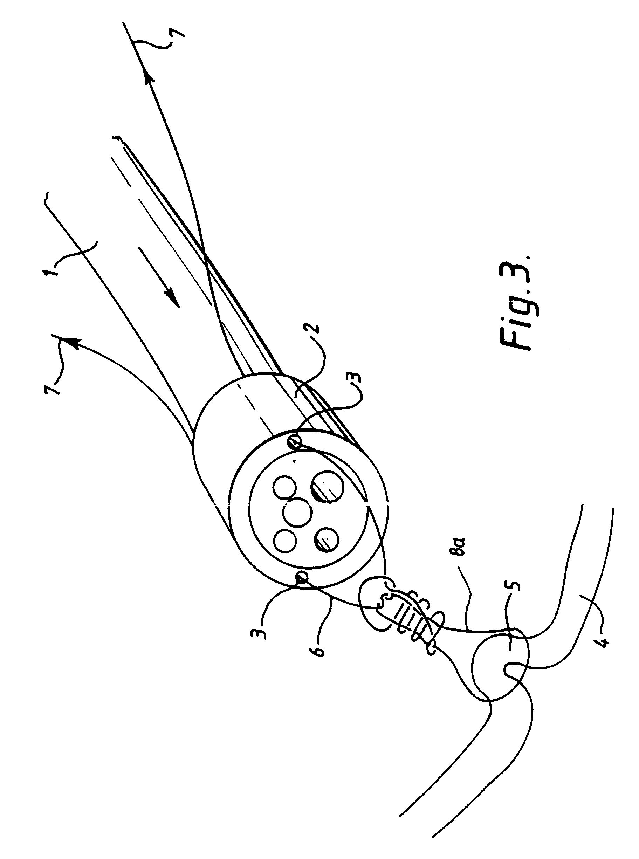

The way in which the arrangement shown in Figure 1 is used in practice can be seen from Figures 2 and 3.

Figure 2 additionally shows part of a patient's tissue 4, including a U-shaped tissue portion 5 through which a thread 6 has been passed by the surgeon. It is desired to tie a knot 8 in the thread 6. At the start of the knot-tying procedure, the ends 7 of the thread 6 are outside the patient, generally emerging through the mouth or anus. Either before or after the thread guide device 2 is mounted on the endoscope 1, a loose knot is tied outside the patient, using the ends 7, and each of the ends 7 is then passed through a respective one of the passages 3 in a direction from the distal end of the endoscope to the proximal end thereof.

The endoscope with the thread guide device thereon is then introduced into the patient and moved towards the portion of tissues. As this is done the loose knot 8 slides along the thread ends until the distal end of the endoscope is adjacent the portion of tissue 5. The surgeon then pulls on the ends 7 in the direction of the arrows indicated thereon. This causes a force to be exerted on the loose knot 8 which has a lateral component and which therefore tends to tighten the knot. During this whole process the knot can be viewed through the viewing channel of the endoscope, since the knot is directly in front of the viewing channel of the endoscope, and the field of view is not in any way impaired by the thread guide device 2.

The process just described serves to tie a single half hitch knot. It is common practice for a surgical knot to consist of more than one half hitch, say two or three, and this can be achieved using the arrangement shown in Figure 2, with the endoscope 1 and thread guide device 2 being withdrawn from the patient and reinserted each time an additional half hitch is made.

Figure 3 shows the use of the same arrangement as Figure 2, but to tie a self-lock slipknot 8a. Apart from the differences in the knots themselves, the procedure used is the same as that just described with reference to Figure 2.

The thread guide device shown in Figures 1 to 3 is a component which is distinct from the endoscope itself, and is removable therefrom. However, it would alternatively be possible for the device to be integral with, or permanently secured to, the endoscope.

## Claims

1. An endoscope (1) having a distal end and a proximal end, and a thread guide device in the form of an annular member (2) which surrounds the said distal end without impairing the field of view of the endoscope, the thread guide device having a pair of thread guide means (3) which are laterally spaced from one another, the device permitting a knot formed by threads which have passed through the thread guide means to be viewed by the endoscope.

2. An endoscope according to claim 1, wherein the thread guide (3) means are diametrically opposed to one another on opposite sides of the endoscope.

3. An endoscope according to claim 1 or 2, wherein the said annular member (2) is cylindrical.

4. An endoscope according to any preceding claim, wherein each of the thread guide means (3) is in the form of a passage extending through the annular member (2).

5. An endoscope according to claim 4, wherein the said passages are parallel to one another and to the axis of rotational symmetry of the annular member (2).

## Patentansprüche

1. Endoskop (1) mit einem distalen Ende und einem proximalen Ende, und einer Fadenführungsvorrichtung in der Form eines ringförmigen Elements (2), das das distale Ende ohne Beeinträchtigung des Gesichtsfeld des Endoskops umgibt, wobei die Fadenführungsvorrichtung ein Paar Fadenführungsmittel (3) aufweist, die seitlich voneinander beabstandet sind, und die Vorrichtung es erlaubt, einen durch die durch die Fadenführungsvorrichtung gelaufenen Fäden gebildeten Knoten durch das Endoskop zu betrachten.

2. Endoskop nach Anspruch 1, dadurch gekennzeichnet, daß die Fadenführungsmittel (3) sich an entgegengesetzten Seiten des Endoskops diametral gegenüberliegen.

3. Endoskop nach Anspruch 1 oder 2, dadurch gekennzeichnet, daß das ringförmige Element (2) zylindrisch ist.

4. Endoskop nach mindestens einem der vorhergehenden Ansprüche, dadurch gekennzeichnet, daß jedes der Fadenführungsmittel (3) in der Form eines Durchlasses vorliegt, der sich durch das ringförmige Element (2) erstreckt.

5. Endoskop nach Anspruch 4, dadurch gekennzeichnet, daß die Durchlässe parallel zueinander und zur Achse der Rotationssymmetrie des ringförmigen Elements (2) sind.

## Revendications

1. Endoscope (1) ayant une extrémité distale et une extrémité proximale, et un dispositif guide-fil sous la forme d'un élément annulaire (2) qui entoure ladite extrémité distale, sans affecter le champ de vision de l'endoscope, le dispositif guide-fil comprenant une paire de moyens de guide-fil (3) qui sont espacés latéralement l'un de l'autre, le dispositif permettant à un noeud formé par des fils qui ont été enfilés dans les moyens de guide-fil d'être visualisé par l'endoscope.

2. Endoscope selon la revendication 1, dans lequel les moyens de guide-fil (3) sont diamétralement opposés l'un à l'autre, sur des côtés opposés de l'endoscope.

3. Endoscope selon la revendication 1 ou 2, dans lequel ledit élément annulaire (2) est cylindrique.

4. Endoscope selon l'une quelconque des revendications qui précèdent, dans lequel chacun des moyens de guide-fil (3) est sous la forme d'un passage traversant l'élément annulaire (2).

5. Endoscope selon la revendication 4, dans lequel lesdits passages sont parallèles l'un à l'autre et à l'axe de symétrie rotationnelle de l'élément annulaire (2).
